# EUROPÄISCHE PATENTSCHRIFT

(11) **EP 0 842 929 B1**
(45) Veröffentlichungstag und Bekanntmachung des Hinweises auf die Patenterteilung: **26.07.2000**
(21) Anmeldenummer: 97120017.5
(22) Anmeldetag: 14.11.1997
(51) Int. Cl.: C07D 317/20

(54) **Verfahren zur Herstellung von cyclischen Acetalen oder Ketalen**
Process for the preparation of cyclic acetals or ketals
Procédé pour la préparation des acétales et kétones cycliques

(30) Priorität: 15.11.1996 DE 19647395
(43) Veröffentlichungstag der Anmeldung: 20.05.1998
(73) Patentinhaber: BASF AKTIENGESELLSCHAFT, 67056 Ludwigshafen (DE)
(72) Erfinder: Bruchmann, Bernd, 67251 Freinsheim (DE); Häberle, Karl, 67346 Speyer (DE); Gruner, Helmut, 01987 Schwarzheide (DE); Hirn, Michael, 68165 Mannheim (DE)
(74) Vertreter: Kinzebach, Werner, Dr.

(56) Entgegenhaltungen:
- EP-B- 0 456 073
- CHEMICAL ABSTRACTS, vol. 97, no. 7, 16.August 1982 Columbus, Ohio, US; abstract no. 55794v, G. PEREZ ET. AL.: "Isopropylidene glycerol." Seite 650; Spalte 2; XP002055983 & ES 499 129 A (CALIPE S.A.)
- CHEMICAL ABSTRACTS, vol. 76, no. 23, 5.Juni 1972 Columbus, Ohio, US; abstract no. 139926u, J. CECHINKI ET. AL. : "Isopropylideneglycerol" Seite 400; Spalte 1; XP002055984 & PL 63 823 A
- C. J. LACEY ET. AL.: "Phospholipid Synthesis Based on New Sequential Phosphate and Carboxylate Ester Bond Formation Steps." JOURNAL OF ORGANIC CHEMISTRY, Bd. 48, Nr. 26, 30.Dezember 1983, Seiten 5214-21, XP002055981
- J. KEMPE: "Herstellung von 2,2-Dimethyl-4-methylol-1,3-dioxolan." ZEITSCHRIFT FÜR CHEMIE, Bd. 26, Nr. 3, März 1986, Seiten 97-8, XP002055982

## Beschreibung

Die Erfindung betrifft ein Verfahren zur Herstellung von cyclischen Acetalen und Ketalen durch Umsetzung eines Polyols mit einem Überschuß eines Aldehyds oder Ketons mit 1 bis 6 Kohlenstoffatomen.

Die Herstellung von cyclischen Acetalen, z.B. von 2,2-Dimethyl-4-methylol-l,3-dioxolan, durch Umsetzung von z.B. Glycerin mit Aldehyden (z.B. Formaldehyd) oder Ketonen (z.B. Aceton oder Methylethylketon) unter Säurekatalyse ist seit langem bekannt. Problematisch ist bei jedem Herstellungsprozeß die Entfernung des Reaktionswassers und die Vermeidung einer intermolekularen Reaktion der Alkohole mit den Aldehyden oder Ketonen, die zu einem Aufbau von Oligomeren führt und damit die Ausbeute an cyclischen Acetalen vermindert (siehe hierzu Literaturstelle [1]).

Es sind sowohl diskontinuierliche als auch kontinuierliche Produktionsverfahren üblich, wobei die diskontinuierlichen überwiegen. So wird in der Literaturstelle [2] ein diskontinuierliches Verfahren zur Herstellung von Isopropylidenglycerin (IPG) aus Glycerin und Aceton im molaren Verhältnis von 1:1,1 unter Zugabe eines sauren Katalysators ohne Wasserschlepper beschrieben. Bei diesem Verfahren reagieren Glycerin und Aceton bis zur Gleichgewichtskonzentration. Anschließend wird erhitzt und das Reaktionswasser bei 0,01333 bar (10 Torr) entfernt. Danach wird der Rückstand bei 0,00667 bar (5 Torr) destilliert, um das gewünschte Produkt zu gewinnen. Die angegebene Ausbeute an IPG liegt bei 80%. Dieses Verfahren weist den Nachteil auf, daß die Ausbeute an IPG niemals über die durch das Reaktionsgleichgewicht vorgegebene Konzentration hinausgehen kann. Bei dem angegebenen Verhältnis von Aceton zu Glycerin ist ebenfalls eine intermolekulare Reaktion in deutlichem Ausmaß zu erwarten. Nachteilig ist auch, daß man eine für Vakuum ausgelegte Apparatur benötigt.

Ein ähnliches Verfahren wird in der Literaturstelle [3] beschrieben. Hierbei werden Glycerin oder Trimethylolpropan (TMP) mit einem Überschuß an Aceton unter Katalyse von p-Toluolsulfonsäure erhitzt. Nach der Neutralisation und Filtration wird das Reaktionsprodukt durch Destillation gereinigt. Die Ausbeuten betragen 56%.

Die Verwendung von Schleppmitteln zur Entfernung des Reaktionswassers ist üblich und wird z.B. in der Literaturstelle [1] beschrieben. Auch in der Literaturstelle [4] wird ganz allgemein ein Verfahren zur Herstellung von cyclischen Ketalen aus Glycerin und n-Alkylmethylketonen, z.B. 2-Butanon oder 2-Hexanon, beschrieben. Dabei werden Glycerin, das entsprechende Keton, Benzol und p-Toluolsulfonsäure zusammen erhitzt. Das Benzol wirkt als Schleppmittel für das entstehende Reaktionswasser. Es wird eine Ausbeute von 85 Mol-% angegeben. Dieses Verfahren ist jedoch bei Verwendung von Aceton als Keton untauglich, da Aceton bereits vor Benzol destillativ abgetrennt und so der eigentlichen Reaktion entzogen wird. Die Ausbeuten an cyclischen Ketalen sind hierbei sehr gering.

Bei der Umsetzung von Glycerin mit Aceton sind daher als Wasserschlepper Petrolether oder auch Chloroform gebräuchlich (siehe Literaturstellen [5] bis [7]). Die Effektivität dieser Lösungsmittel ist indessen nicht besonders hoch, da sie sich gut mit Aceton mischen. Dies beeinträchtigt die Phasentrennung des Wassers mit dem organischen Lösungsmittelgemisch erheblich und führt zu einem unvollständigen Auskreisen des Reaktionswassers, was sich auch in der sehr langen Reaktionszeit (43 h bei Petrolether, Literaturstelle [6] dokumentiert.

Die Entfernung des Reaktionswassers in situ durch Trocknungsmittel wurde ebenfalls beschrieben. In den Literaturstellen [8] und [9] werden Natriumsulfat und Phosphorpentoxid, die gleichzeitig als Katalysatoren wirken, genannt. In neuerer Zeit sind auch Molekularsiebe verwendet worden (Literaturstelle [10]). Diese Verfahren sind für den Produktionsmaßstab ungeeignet, weil das Trocknungsmittel entweder als Abfallprodukt anfällt oder aufwendig regeneriert werden müßte.

Um Probleme der Herstellung von Isopropylidenglycerin im Batch-Prozeß zu umgehen, wurden verfahrenstechnisch aufwendige kontinuierliche Herstellungsverfahren beschrieben. In der Literaturstelle [11] wird die Reaktion von Glycerin mit Aceton unter Säurekatalyse nur bis zur Gleichgewichtskonzentration (etwa 45% IPG) durchgeführt. Nach der Desaktivierung des Katalysators werden Wasser, Aceton, IPG und Glycerin destillativ getrennt, und die nicht umgesetzten Anteile an Glycerin und Aceton werden der Reaktion wieder zugeführt. Der desaktivierte Katalysator bleibt jedoch im Glycerin zurück und kann Nebenreaktionen hervorrufen. In der Literaturstelle [12] wird daher vom gleichen Autor ein saurer Festbett-Ionenaustauscher als Katalysator vorgeschlagen, bei dem eine Neutralisation entfällt. Bei diesen beiden Methoden ([11] und [12]) wird infolge des unvollständigen Umsatzes Glycerin als Sumpfprodukt in der Destillation thermisch sehr belastet. Dies führt zu Nebenprodukten, die in einem kontinuierlichen Prozeß ausgeschleust werden müssen und so die Ausbeuten beträchtlich verringern können. Wegen der Aufarbeitung der Produkte im Vakuum ist außerdem eine aufwendige Reaktionsführung nötig.

Der vorliegenden Erfindung liegt die Aufgabe zugrunde, ein Verfahren zur Herstellung von cyclischen Acetalen oder Ketalen bereitzustellen, das einfach und auch mit Aldehyden oder Ketonen durchführbar ist, die zumindest teilweise mit Wasser mischbar sind und/oder einen Siedepunkt aufweisen, der im Bereich des Siedepunktes von Wasser oder darunter liegt.

Überraschenderweise wurde nun gefunden, daß diese Aufgabe gelöst wird, wenn man den Aldehyd oder das Keton im Überschuß einsetzt und den Aldehyd oder das Keton während der Reaktion abdestilliert und den abdestillierten Aldehyd oder das abdestillierte Keton durch frischen bzw. frisches ersetzt.

Gegenstand der vorliegenden Erfindung ist daher ein, vorzugsweise diskontinuierliches, Verfahren zur Herstellung von cyclischen Acetalen oder Ketalen durch Umsetzung eines Polyols mit einem Überschuß eines Aldehyds oder eines Ketons mit 1 bis 6 Kohlenstoffatomen, das dadurch gekennzeichnet ist, daß man während der Umsetzung Aldehyd oder Keton abdestilliert und den abdestillierten Aldehyd oder das abdestillierte Keton durch frischen bzw. frisches ersetzt.

Brauchbare Aldehyde oder Ketone (im folgenden als Carbonylverbindungen bezeichnet) sind insbesondere solche, deren Siedepunkt bei Normaldruck < 110°C und vorzugsweise < 100°C ist. Besonders bevorzugt sind Carbonylverbindungen mit 1 bis 4 Kohlenstoffatomen und insbesondere Aceton, 2-Butanon, Formaldehyd, Acetaldehyd oder Propionaldehyd.

Überraschenderweise hat sich gezeigt, daß die Carbonylverbindung nicht nur Reaktionskomponente ist, sondern auch als Transportmedium für das bei der Umsetzung entstehende Wasser dient, obwohl die Carbonylverbindungen zumindest teilweise oder sogar vollständig (wie Aceton, Methylethylketon, Formaldehyd, Acetaldehyd) mit Wasser mischbar sind und unter den Destillationsbedingungen kein Azeotrop bilden. Trotzdem wird auf diese Weise das gebildete Wasser aus dem Gleichgewicht entfernt, so daß hohe Ausbeuten an cyclischen Verbindungen erzielt werden.

Bei dem erfindungsgemäßen Verfahren wird die bei der Umsetzung abdestillierte Carbonylverbindung durch frische, d.h. durch eine Carbonylverbindung ersetzt, deren Wassergehalt ≤ 1% und insbesondere ≤ 0,8% ist. Der Ersatz der Carbonylverbindung kann durch stufenweise Zugabe oder vorzugsweise kontinuierliche Zugabe in das Reaktionsgemisch erfolgen. Dies erfolgt zweckmäßigerweise entsprechend der Verdampfungsrate, d.h. die Menge an Carbonylverbindung in der Reaktionsmischung wird konstant gehalten. Die abdestillierte Carbonylverbindung kann in üblicher Weise, beispielsweise durch Destillation oder durch Absorption des Wassers an Trocknungsmitteln, getrocknet und wiederverwendet werden.

Vorzugsweise destilliert man die Carbonylverbindung nicht während der gesamten Dauer der Umsetzung ab, sondern man beginnt mit dem Abdestillieren erst, nachdem sich eine wesentliche Menge des Acetals oder Ketals gebildet hat. Zweckmäßigerweise beginnt man, sobald sich das Reaktionsgleichgewicht eingestellt hat.

Die Menge an Carbonylverbindung kann in einem weiten Bereich variieren. Im allgemeinen verwendet man einen Überschuß von mindestens 10 Mol-%, bezogen auf die Menge an Polyol. Vorzugsweise verwendet man 2 bis 30 Mol, insbesondere 3 bis 15 Mol Carbonylverbindung pro Mol Polyol.

Als Polyol sind alle Verbindungen brauchbar, die mindestens zwei Hydroxygruppen in einer Position aufweisen, die zur Bildung eines cyclischen Acetals oder Ketals mit 5 bis 8 und insbesondere 5 oder 6 Ringatomen führt. Brauchbare Polyole sind:
- aliphatische Diole, deren Hydroxygruppen in 1,2-, 1,3-, 1,4- oder 1,5-Position stehen. Beispiele hierfür sind Ethylenglykol, 1,2-Propylenglykol, 1,3-Propylenglykol, 1,2-Butandiol, 1,3-Butandiol, 1,4-Butandiol, 1,5-Pentandiol, Neopentylglykol etc.;
- aromatische Diole, wie 1,2-Dihydroxybenzol;
- aliphatische Triole, wie Glycerin, Trimethylolpropan, Trimethylolethan oder Trimethylolmethan;
- aliphatische Tetrole, wie Pentaerythrit;
- Zuckeralkohole mit 4 bis 6 Hydroxygruppen, wie Threit, Erythrit, Xylit, Dulcit, Mannit und Sorbit;
- Zucker, wie Aldosen und Ketosen, beispielsweise Glucose, Mannose, Fructose, etc.

Bevorzugt sind Diole oder Triole mit 2 bis 12 Kohlenstoffen, insbesondere Ethylenglykol, 1,2- und 1,3-Propylenglykol, Glycerin und Trimethylolpropan.

Die Reaktionstemperatur liegt im allgemeinen im Bereich von ca. 20°C bis zur Siedetemperatur des Reaktionsgemisches. Die Reaktionszeit richtet sich nach der Reaktionstemperatur und den Mengenverhältnissen der Ausgangsprodukte. Sie beträgt im allgemeinen ca. 20 Min bis 48 h.

Alle zur Herstellung von Acetalen oder Ketalen brauchbaren Katalysatoren können zur Anwendung kommen, z.B. Säuren, wie Toluolsulfonsäure, Schwefelsäure, Chlorwasserstoff, saure Ionenaustauscher etc.

Die Menge an Katalysator beträgt im allgemeinen 0,01 bis 0,5 Mol-%, bezogen auf den zur Anwendung kommenden Alkohol.

Nach dem erfindungsgemäßen Verfahren werden die intramolekularen Reaktionsprodukte, nämlich cyclische Acetale bzw. Ketale, in hohen Reinheitsgraden, nämlich von 95 bis 99,5%, erhalten. Für viele Anwendungszwecke sind diese Reinheitsgrade bereits ausreichend, so daß sich, speziell bei der Herstellung von Isopropylidenglycerin, eine weitere Aufarbeitung des Reaktionsproduktes, z.B. durch Destillation, erübrigt.

Die nachfolgenden Beispiele erläutern die Erfindung, ohne sie zu begrenzen.

### Beispiele

1 mol Alkohol und 4 mol Keton (entsprechend Tabelle 1) wurden vorgelegt, und 0,05 Mol-% p-Toluolsulfonsäure-Monohydrat, bezogen auf Alkohol, wurden zugegeben. Die Reaktionsmischung wurde 30 Minuten unter Rückfluß erhitzt. Anschließend wurde Keton destillativ entfernt und der Flüssigkeitsstand des Reaktors synchron durch den kontinuierlichen Zulauf von trockenem Keton konstant gehalten. Der Reaktionsfortschritt wurde mittels Gaschromatographie verfolgt. Die Reaktion wurde nach Erreichen des gewünschten Umsatzes durch Zugabe von 0,1 Mol-% (bezogen auf Alkohol) Natriummethanolat abgebrochen. Anschließend wurde das überschüssige Keton destillativ entfernt.

Die Reinheit der Endprodukte wurde gaschromatographisch bestimmt. Die Reaktionszeiten sowie weitere Daten zu den Endprodukten gehen aus Tabelle 1 hervor. Die Zulaufmenge des trockenen Ketons wurde in den Beispielen gewichtsmäßig mit etwa 8 bis 15 Teilen Keton auf 1 Teil des eingesetzten Alkohols bestimmt.

**Tabelle 1**

| (erfindungsgemäße Beispiele) | | | | |
|---|---|---|---|---|
| Beispiel | Alkohol | Keton | Reaktionszeit (h) | Ausbeute Endprodukt (%) |
| 1 | Glycerin | Aceton | 9 | 97,6 |
| 2 | Glycerin | Aceton | 12 | 99,5 |
| 3 | Glycerin | 2-Butanon | 12 | 97,0 |
| 4 | Trimethylolpropan | Aceton | 9 | 98,7 |
| 5 | Ethylenglykol | Aceton | 8 | 99,1 |

### Vergleichsbeispiele 1 und 2 (in Anlehnung an Literaturstelle [6])

1. 552 g (6 mol) Glycerin, 1392 g (24 mol) Aceton, 1400 g Petrolether (Siedebereich 30 bis 75°C) und 0,47 g (0,05 Mol-%, bezogen auf Glycerin) p-Toluolsulfonsäure-Monohydrat wurden zusammengegeben und bis zum Siedepunkt der Lösungsmittel erhitzt. Mittels eines Wasserauskreisers wurde das Reaktionswasser entfernt. Nach 42 Stunden wurde im Wasserauskreiser kein Wasser mehr abgeschieden. Die Reaktion wurde durch Zugabe von 0,94 g Natriummethanolat gestoppt, und die Lösungsmittel wurden im Vakuum am Rotationsverdampfer entfernt. Das Reaktionsgemisch enthielt das gewünschte Produkt in einer Ausbeute von 92,5%.
2. 792 g (6 mol) Trimethylolpropan, 1392 g (24 mol) Aceton, 1400 g Petrolether (Siedebereich 30 bis 75°C) und 0,47 g p-Toluolsulfonsäure-Monohydrat wurden zusammengegeben und bis zum Siedepunkt der Lösungsmittel erhitzt. Mittels eines Wasserauskreisers wurde das Reaktionswasser entfernt. Nach 14 Stunden wurde im Wasserauskreiser kein Wasser mehr abgeschieden. Die Reaktion wurde durch Zugabe von 0,94 g Natriummethanolat gestoppt, und die Lösungsmittel wurden im Vakuum am Rotationsverdampfer entfernt. Das Reaktionsgemisch enthielt das gewünschte Produkt in einer Ausbeute von 91,5%.

### Literatur

[1] J. Kempe und G. Kiessling, Z. Chem. 26 (1986)3, 97
[2] ES 499 129 A1
[3] EP 456 073 B1
[4] A. Piasecki, Polish Journal of Chemistry 58 (1984), 1215
[5] J. Rübner und H. Frommelt, Mitteilungsbl. Chem. Ges. DDR 31 (1984), 56
[6] M.S. Newman und N. Renoll, J. Amer. Chem. Soc. 67 (1945), 1621
[7] PL 63 823
[8] E. Fischer und E. Pfähler, Ber. Dt. Chem. Ges. 53 (1920), 1027
[9] R. Aldo Macchi, T. Crespo, Rev. Argent. Gras. Aceites 9 [2] (1972), 9
[10] Meng Shen Cai et al., Synth. Commun. 22 (1992) 18,2653
[11] DD 238 232 A1
[12] DD 238 233 A1

## Patentansprüche

1. Verfahren zur Herstellung von cyclischen Acetalen oder Ketalen durch Umsetzung eines Polyols mit einem Überschuß eines Aldehyds oder eines Ketons mit 1 bis 6 Kohlenstoffatomen, dadurch gekennzeichnet, daß man während der Umsetzung einen Teil des Aldehyds oder Ketons abdestilliert und den abdestillierten Aldehyd oder das abdestillierte Keton durch frischen bzw. frisches ersetzt.

2. Verfahren nach Anspruch 1, dadurch gekennzeichnet, daß man als Polyol ein Diol oder Triol mit 2 bis 12 Kohlenstoffatomen, insbesondere Ethylenglykol, Propylenglykol, Glycerin oder Trimethylolpropan, verwendet.

3. Verfahren nach einem der vorhergehenden Ansprüche, dadurch gekennzeichnet, daß man einen Aldehyd oder ein Keton verwendet, dessen Siedepunkt bei Normaldruck < 110°C und insbesondere < 100°C ist.

4. Verfahren nach Anspruch 3, dadurch gekennzeichnet, daß man als Keton Aceton oder 2-Butanon verwendet.

5. Verfahren nach einem der vorhergehenden Ansprüche, dadurch gekennzeichnet, daß man 2 bis 30 Mol Aldehyd oder Keton pro Mol Polyol einsetzt.

6. Verfahren nach einem der vorhergehenden Ansprüche, dadurch gekennzeichnet, daß man die Umsetzung in Gegenwart eines sauren Katalysators durchführt.

## Claims

1. A process for preparing cyclic acetals or ketals by reacting a polyol with an excess of an aldehyde or a ketone having 1 to 6 carbon atoms, wherein part of the aldehyde or ketone is distilled out during the reaction and the aldehyde or ketone distilled out is replaced by fresh aldehyde or ketone.

2. A process as claimed in claim 1, wherein a diol or triol having 2 to 12 carbon atoms, in particular ethylene glycol, propylene glycol, glycerol or trimethylolpropane, is used as polyol.

3. A process as claimed in either of the preceding claims, wherein an aldehyde or ketone whose boiling point under atmospheric pressure is < 110°C and, in particular, < 100°C is used.

4. A process as claimed in claim 3, wherein acetone or 2-butanone is used as ketone.

5. A process as claimed in any of the preceding claims, wherein from 2 to 30 mol of aldehyde or ketone are employed per mole of polyol.

6. A process as claimed in any of the preceding claims, wherein the reaction is carried out in the presence of an acidic catalyst.

## Revendications

1. Procédé pour la préparation d'acétals ou cétals cycliques par réaction d'un polyol avec un excès d'un aldéhyde ou d'une cétone en C1-C6, caractérisé par le fait que, dans le cours de la réaction, on élimine par distillation une partie de l'aldéhyde ou de la cétone et on remplace l'aldéhyde ou la cétone distillée par un aldéhyde frais ou respectivement une cétone fraîche.

2. Procédé selon la revendication 1, caractérisé par le fait que le polyol utilisé est un diol ou triol en C2-C12, plus particulièrement l'éthylèneglycol, le propylèneglycol, le glycérol ou le triméthylolpropane.

3. Procédé selon une des revendications qui précèdent, caractérisé par le fait que l'on utilise un aldéhyde ou une cétone dont le point d'ébullition à pression normale est inférieur à 110°C et plus spécialement à 100°C.

4. Procédé selon la revendication 3, caractérisé par le fait que la cétone utilisée est l'acétone ou la 2-butanone.

5. Procédé selon une des revendications qui précèdent, caractérisé par le fait que l'on met en oeuvre 2 à 30 mol de l'aldéhyde ou de la cétone par mole du polyol.

6. Procédé selon une des revendications qui précèdent, caractérisé par le fait que l'on précède à la réaction en présence d'un catalyseur acide.
